# EUROPEAN PATENT APPLICATION

(11) **EP 3 560 523 A1**
(43) Date of publication of application: **30.10.2019**
(21) Application number: 19168878.7
(22) Date of filing: 12.04.2019
(51) Int. Cl.: A61L 9/12

(54) **NEW AIR FRESHENER DISPENSER FOR CAR**

(30) Priority: 27.04.2018 ES 201830612 U
(71) Applicant: Rodriguez-Barbero Gonzalez, Ana Isabel, 28939 Madrid (ES)
(72) Inventor: Rodriguez-Barbero Gonzalez, Ana Isabel, 28939 Madrid (ES)
(74) Representative: Lorente Berges, Ana

(57) **Abstract**

The present invention discloses a new air freshener dispenser (1) for car, comprising an air freshener container (2) and a cap (3) couplable to an open upper end of said air freshener container (2). The cap (3) is made of a porous material allowing for a controlled evaporation of the air freshener contained in the container (2). In particular, the cap (3) of the air freshener dispenser (1) of the invention is made of a ceramic material, e.g. a mix of natural gypsum, plaster, sand and kaolin.

## Description

### OBJECT OF THE INVENTION

The present invention belongs to the field of air freshener dispensing in vehicles.

The object of the present invention is a new dispenser having a cap made of a ceramic material providing for a gradual evaporation of the air freshener.

### PRIOR ART

Nowadays, a plurality of air freshener dispensers designed for providing a nice fragrance in a vehicle are known. These dispensers usually comprise a container, made of glass or a plastic material, where the air freshener in a liquid state is contained and a cap that is couplable to an open upper end of said container. The cap may contain an air freshener dosing means which may have several forms according to the application.

Nowadays, a dispenser of this type where the dosing is carried out by means of the cap having a porosity level allowing for the air freshener contained in the container to slowly evaporate is known. The evaporation speed of the air freshener can be controlled by the porosity leve lof the material making up the dispenser cap. Usually, the cap is made of wood.

A drawback of this type of dispensers is that they don't allow for a prolonged use going further than the initial charge they have when they are sold. Indeed, the absorbing properties of wood and its tendency to swell with moisture cause the cap to eventually become saturated with air freshener. When saturation occurs, the wood loses its porosity characteristic and the air freshener no longer can evaporate through said cap. Consequently, once one charge of air freshener is used up, a recharge for enjoying the air freshener fragrance using the same dispenser is not possible.

### DESCRIPTION OF THE INVENTION

The present invention discloses a new air freshener dispenser for vehicles that solves the aforementioned drawbacks.

According to the present invention, the air freshener dispenser for vehicles comprises an air freshener container and a cap that is couplable to an open upper end of said air freshener container. As mentioned above, the cap is made of a porous material allowing for a controlled evaporation of the air freshener contained in the container. However, unlike the known dispensers, the dispenser of the invention is characterized in that que cap is made of a ceramic material.

Indeed, in the present invention the wood usually employed for producing the cap is replaced by a porous ceramic material. Ceramic material does not have the characteristics of wood in connection with the absorption capacity or the tendency to swell with moisture. On the contrary, a porous ceramic material does not swell or absorb the air freshener; the air freshener merely passes through the pores of the ceramic cap at a rate directly depending on the pore size. That is, the ceramic cap used in the present invention does not saturate as the air freshener evaporates therethrough. Therefore, the use of a ceramic material allows the user to refill the container of the dispenser as many times as needed, thereby solving the problem of the prior art.

According to a particularly preferred embodiment of the invention, the ceramic material used for the cap of the dispenser of the present invention comprises a mix of natural gypsum, plaster, sand and kaolin. This composition allows for obtaining a ceramic material having a high hardness and, at the same time, having optimal porosity characteristics for the diffusion of the air freshener.

According to another preferred embodiment of the invention, the ceramic cap has a female thread that is couplable to a male thread present at the upper open end of the container. This is important in that it allows for a fast and simple coupling and uncoupling of the cap to/from the container.

In still another preferred embodiment of the invention, un upper end of the ceramic cap comprises a through hole. This hole allows for the passage of a rope, cord or string for hanging the dispenser of the present invention, for example from the rear view mirror of the vehicle.

In still one more preferred embodiment of the invention, a lower end of the container comprises a flat surface. This flat surface allows for the dispenser of the invention to stand on a horizontal surface, such as a table or the like.

### BRIEF DESCRIPTION OF THE DRAWINGS

Fig. 1 shows a perspective view of a dispenser according to the present invention.
Fig. 2 shows another perspective view of the dispenser according to the present invention.
Fig. 3 shows one more perspective view of the dispenser of the invention where the cap is not coupled to the container.
Fig. 4 shows another perspective view of the cap of the dispenser according to the present invention.

### PREFERRED EMBODIMENT OF THE INVENTION

A particular example of an air freshener dispenser (1) according to the present invention is now disclose making reference to the attached drawings.

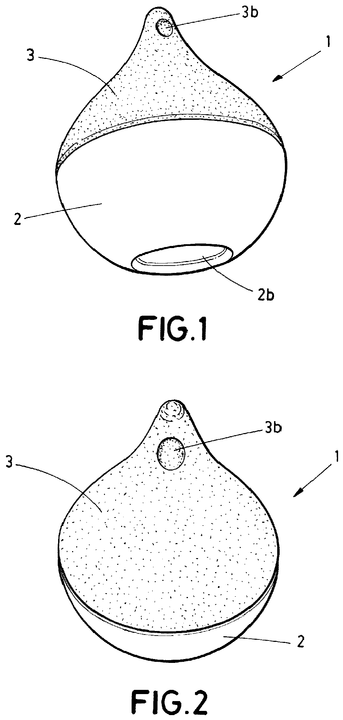

Figs. 1 and 2 show respective perspective views of the air freshener dispenser (1) according to the present invention where the component parts are visible. Specifically, the dispenser (1) has a translucent glass container (2) having an essentially spherical shape except for a flattening (2b) provided at the base for allowing it to stand on a flat surface, such as a table or a car dashboard. The container (2) is coupled to a cap (3) made of a porous ceramic material made of natural gypsum, plaster, sand and kaolin having an essentially frustoconical shape. The upper and of the cap (3) comprises a through hole (3b) allowing for the passage of a rope, cord or string for hanging the dispenser (1) from the rear view mirror of a vehicle.

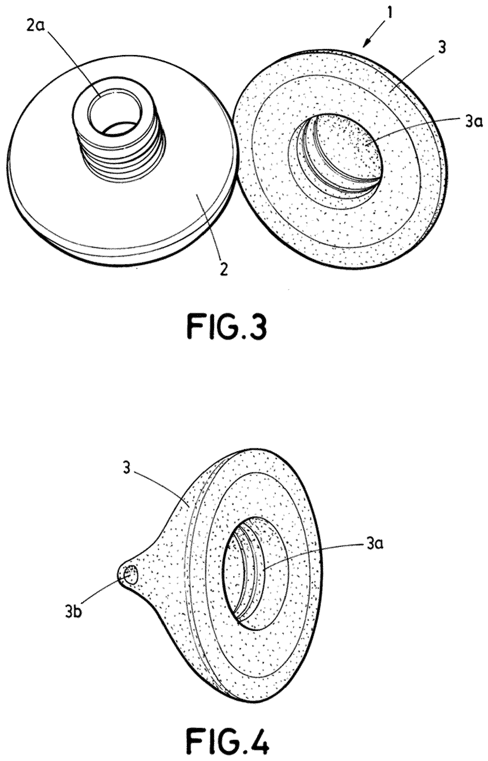

Fig. 3 shows the container (2) and the cap (3) in an uncoupled state. The male thread (2a) of the container (2) and the complementary female thread (3a) of the cap (3) are visible. Fig. 4 shows another view of the cap (3) where the female thread (3a) is visible.

## Claims

1. New air freshener dispenser (1) for car, comprising an air freshener container (2) and a cap (3) couplable to an open upper end of said air freshener container (2), where the cap (3) is made of a porous material allowing for a controlled evaporation of the air freshener contained in the container (2), **characterized in that** the cap (3) is made of a ceramic material.

2. New dispenser (1) according to claim 1, where the ceramic material comprises a mix of natural gypsum, plaster, sand and kaolin.

3. New dispenser (1) according to any of the previous claims, where the ceramic cap (3) has a female thread (3a) couplable to a male thread (2a) present at the upper open end of the container (2).

4. New dispenser (1) according to any of the previous claims, where an upper end of the ceramic cap (3) comprises a through hole (3b).

5. New dispenser (1) according to any of the previous claims, where a lower end of the container (2) comprises a flat surface (2b).
